# EUROPÄISCHE PATENTSCHRIFT

(11) **EP 0 456 152 B1**
(45) Veröffentlichungstag und Bekanntmachung des Hinweises auf die Patenterteilung: **04.11.1998**
(21) Anmeldenummer: 91107307.0
(22) Anmeldetag: 06.05.1991
(51) Int. Cl.: C07K 7/06, G01N 33/86, A61K 38/08

(54) **Peptidamide, Verfahren zu deren Herstellung und diese enthaltende Mittel als Fibrin/Thrombin-Gerinnungsinhibitoren**
Peptidamides, their process of preparation and the agents containing these peptides as fibrin-thrombin clotting inhibitors
Peptide-amides, leur procédé de préparation et les agents contenant ces peptides inhibiteurs de la coagulation de fibrine/thrombine

(30) Priorität: 08.05.1990 DE 4014655
(43) Veröffentlichungstag der Anmeldung: 13.11.1991
(73) Patentinhaber: Dade Behring Marburg GmbH, 35001 Marburg (DE)
(72) Erfinder: Stüber, Werner, Dr., W-3551 Lahntal (DE); Fickenscher, Karl, Dr., W-3550 Marburg (DE)

(56) Entgegenhaltungen:
- JOURNAL OF MEDICINAL CHEMISTRY. Bnd. 24, Nr. 3, 1. März 1981, WASHINGTON US Seiten 322 - 327; KH HSIEH ET AL.: 'fibrin polymerization. 1. alkylating peptide inhibitors of fibrin polymerization'
- CHEMICAL ABSTRACTS, Bnd. 113, Nr. 19, 5. November 1990, Columbus, Ohio, US; Zusammenfassung 172426M, K KAWASAKI ET AL.: 'proparation of antithrombotic proline-containing peptideamides' Seite 744, Spalte L;
- CHEMICAL ABSTRACTS, Bnd. 107, Nr. 23, 7. Dezember 1987, Columbus, Ohio, US; Zusammenfassung 215362H, N G LEVITSKAYA ET AL.: 'the influence of low molecular weight synthetic peptides on fibrin formation' Seite 389, Spalte R;
- CHEMICAL ABSTRACTS, Bnd. 115, Nr. 11, 16. September 1991, Columbus Ohio, US; Zusammenfassung 109014B, K FICKENSCHER ET AL.: 'a photometric assay or blood coagulation factor XIII' Seite 371, Spalte L;

## Beschreibung

Die Erfindung betrifft Oligopeptidamide sowie Verfahren zu deren Herstellung. Die beschriebenen Verbindungen sind im Stande, die Clotbildung im Blut, d. h. die Blutgerinnung, zu verhindern. Dadurch sind diese Peptide therapeutisch und diagnostisch von Interesse.

Nach dem Stand des Wissens sind Verbindungen bekannt, die in der Lage sind, die Blutgerinnung zu inhibieren. Solche Substanzen sind unter anderem Peptidderivate und Proteine. Hierzu zählen insbesondere das therapeutisch eingesetzte Antithrombin III und die diagnostisch eingesetzten Peptidchlormethylketone. Die Wirksamkeit dieser Substanzen liegt in der Inhibition von Thrombin, das bedeutet, Fibrinogen wird nicht mehr zu Fibrin abgebaut und F XIII wird nicht aktiviert.
Für diverse diagnostische Zwecke ist es allerdings von Interesse, die Blutgerinnungskaskade unter Bildung von Thrombin voll zu aktivieren, oder direkt Thrombin zum Testansatz zuzusetzen, ohne jedoch ein Gerinnsel entstehen zu lassen. Das bedeutet, es müssen Gerinnungsinhibitoren eingesetzt werden, die in der Lage sind, bereits durch Thrombin gebildetes Fibrin an der Ausbildung eines Gerinnsels zu hindern.
Für therapeutische Zwecke ist es von Interesse, die Ausbildung eines Fibringerinnsels bei Vorhandensein von löslichem Fibrin zu unterbinden. Hierzu zählen z. B. die Verhinderung der Okklusion der Arteriolen bei einer disseminierten intravasalen Gerinnung oder die Unterbindung einer Neuausbildung eines Fibringerinnsels bei einer Lysetherapie unter Fortbestehen der lokalen Gerinnungsaktivierung.

Wie in der Patentschrift DE 38 11 647 gezeigt wurde, kann durch Zusatz eines Gerinnungsinhibitors ein Testsystem für den Blutgerinnungsfaktor XIII aufgebaut werden. Der zugesetzte Clotinhibitor inhibiert nicht das Thrombin, sondern verhindert die Zusammenlagerung von löslichen Fibrinketten. Das verwendete Peptid hat die Peptidsequenz Gly-Pro-Arg-Pro. Allerdings ist von Nachteil, daß von diesem Peptid eine verhältnismäßig große Menge zur vollständigen Gerinnselvermeidung zugesetzt werden muß. Dasselbe trifft auch auf den therapeutischen Einsatz dieses Tetrapeptids zu.

Auf dem XI. Amerikanischen Peptidsymposium (July 1989) wurde eine Reihe weiterer wirksamer Peptidderivate präsentiert (Abkürzungen sind später erläutert):

| **Struktur** | **relative Wirksamkeit** (Menge an Plasma, die mit definierter Inhibitormenge inhibiert wird) |
|---|---|
| GPRP | 1 |
| GPRP-4-hydroxypiperidid | 1.2 |
| GPRP-3-methylpiperidid | 1.29 |
| GPRP-NH2 | 3.52 |
| GPRPP-NH2 | 4.56 |

Die Wirkungssteigerung der gezeigten Peptide beruht auf dem Einbau von cyclischen Aminderivaten als C-terminalem Baustein.

Verbindungen vom Typ GPRPP-NH₂ werden auch in der Japanischen Patentschrift JP 2,115,197 beschrieben. Alkylierende Peptidinhibitoren der Fibrinpolymerisation beschreiben HSIEH et al. (J. Med.Chemistry 24 (1981), S. 322 - 327).

Aufgabe der vorliegenden Erfindung war es, gegenüber dem Stand der Technik noch wirksamere Peptide zu finden.

Gegenstand der Erfindung sind deshalb Peptidamide der allgemeinen Formel I

GPRP-X-NH₂ I

wobei G für die Aminosäure Glycin, P für die Aminosäure L-Prolin, R für die Aminosäure L-Arginin, X für Alanin oder Glycin steht.

Die Aminosäuren, deren Stereoform oben nicht definiert wurde, liegen vorzugsweise in der L-Form vor, können jedoch auch in der D-Form vorliegen.

Die Herstellung der erfindungsgemäßen Peptidderivate erfolgt nach an sich bekannten Methoden, wobei für die Synthese der nichtalkylierten Amide die Solid-Phase-Methode eingesetzt wurde. Vorzugsweise wurde dabei vorgegangen wie in Int. Peptide Protein Res. 34, 215-221, 1989 beschrieben.

Die Peptide wurden bevorzugt an 1% quervernetztem Polystyrol-Divinylbenzol-Copolymer, das mit einer säurelabilen Amidankerfunktion derivatisiert war, aufgebaut. Die anfängliche Aminogruppenbildung lag in dem Bereich von 0.3-0.8 mmol/g Harz.
Die Synthese wurde unter repetitiver Kopplung der einzelnen geschützten Aminosäuren vom C- zum N- Terminus des Peptids ausgeführt. Entsprechend ihrer chemischen Struktur sind die Aminosäuren am N-alpha-Stickstoff und gegebenenfalls an der Drittfunktion geschützt. Die Stickstoffunktionen (N-alpha) waren mit der Fmoc-Gruppe, alkoholische Seitengruppen als tert.-Butylether-, Carboxylgruppen in der Seitenkette als tert.-Butylester und die Guanidingruppe mit der Mtr oder Pmc-Gruppe geschützt.
Der Aufbau der Peptide erfolgte dabei unter Einbezug der folgenden Schritte an einem halb- oder vollautomatischen Peptidsynthesizer:
- Harz waschen (15 ml/g Harz) Lösemittel DMF (oder Dichlormethan oder N-Methylpyrrolidon)
- Abspaltung der Fmoc-Gruppe mit 20% Piperidin in DMF
- Waschen des Harzes mit DMF (oder N-Methylpyrrolidon)
- Ankopplung der Aminosäure durch Einsatz eines Kondensationsmittels wie Carbodiimid, gegebenenfalls unter Zusatz von HOBt oder statt dessen Einsatz eines gemischten oder symmetrischen Anhydrids oder eines Aktivesters.
- nach vollendeter Kopplung Auswaschung der überschüssigen Reagentien (DMF).

Als letzte Aminosäure wurde üblicherweise eine Boc-Aminosäure eingesetzt.
Das Peptid wurde dann mit einer Mischung aus Trifluoressigsäure, vorzugsweise 90%, und in Gegenwart eines Scavengers, wie Ethandithiol, Wasser, Resorcin, Anisol oder Thioanisol, einzeln oder als Scavengermischung während 1-2 Stunden bei Raumtemperatur oder bei Temperaturen bis 40 Grad abgespalten.
Die Peptide wurden durch Präzipitation in einem Ether kristallisiert und durch Gelpermeation gereinigt. Die Reinheit der Peptide wurde durch HPLC und Aminosäureanalyse festgestellt.

Die Synthese der Penta- und Hexapeptidalkylamide wurde nach der klassischen, in Lösung arbeitenden Methode ausgeführt.
Zuerst wurde die geschützte (Boc- oder Z-) Aminosäure an das entsprechende Alkylamin gekoppelt, wobei dieselben Methoden wie bei der Solid-Phase-Methode verwendet wurden. Die Zwischenprodukte wurden, je nach physikalischem Verhalten, durch Umkristallisieren, Ausschütteln und Umfällen aufgereinigt. Nach Abspaltung der N-terminalen Schutzgruppe, im Falle von Boc durch Acidolyse mit 1,2N HCl in Eisessig oder mit 50% TFA in Dichlormethan, im Falle von Z durch Hydrogenolyse, wurde die nächste Aminosäure wie bereits beschrieben angekoppelt. Bei Arginin wurde üblicherweise auf eine Schutzgruppe an der Guanidinogruppe verzichtet, diese war lediglich protoniert.
Nach finaler Entschützung wurden die Peptide wie üblich gereinigt. Dazu kamen als Methode die Gelpermeation und ausnahmsweise auch eine HPLC-Reinigung an RP-18 Material in Frage. Die Verbindungen wurden mit HPLC und Aminosäureanalyse und ¹³C-NMR-Spektroskopie auf Homogenität und Struktur untersucht.
Die erfindungsgemäßen Peptide wurden auch auf ihre Inhibitionsfähigkeit getestet. Hierzu wurde eine Plasmaprobe mit Inhibitor versetzt und nach Zugabe von Thrombin die Zeit bestimmt, bis die Probe geronnen war. Ein repräsentatives Ergebnis ist unter den Beispielen aufgeführt.
Wie man daraus erkennt, haben die erfindungsgemäßen Peptide gegenüber den bekannten Verbindungen deutlich erhöhte Inhibitionspotentiale.
Im Falle der Pentapeptidamide wurde dabei überraschenderweise gefunden, daß als C-terminale Aminosäure besonders kleine Aminosäuren wie Glycin und Alanin günstig sind. Nach dem Stand der Technik werden hier jedoch cyclische Strukturen wie Prolin oder Piperidinderivate bevorzugt.

Abkürzungen:
- Ala = A: Alanin
- Asp = D: Asparaginsäure
- Asn = N: Asparagin
- Gly = G: Glycin
- Val = V: Valin
- Leu = L: Leucin
- Ile = I: Isoleucin
- Ser = S: Serin
- Thr = T: Threonin
- Met = M: Methionin
- Pro = P: Prolin
- Lys = K: Lysin
- Arg = R: Arginin
- Glu = E: Glutaminsäure
- Gln = Q: Glutamin
- Phe = F: Phenylalanin
- Tyr = Y: Tyrosin
- Trp =: W Tryptophan
- HOBt: Hydroxybenzotriazol
- DIC: Diisopropylcarbodiimid
- TFA: Trifluoressigsäure
- Z: Benzyloxycarbonyl
- Boc: Butyloxycarbonyl
- Fmoc: Fluorenylmethyloxycarbonyl
- Pmc: Pentamethylchromansulfonyl
- DMF: Dimethylformamid

Das folgende Beispiel verdeutlicht die Erfindung näher:

### Beispiel:

### Herstellung von Gly-Pro-Arg-Pro-Ala-NH₂

1 g Fmoc-Amidanker-Resin (0.47 mmol Aminogruppen/Gramm) wurde 3 mal mit 15 ml DMF gewaschen und die Fmoc-Gruppe mit 20 % Piperidin in DMF (1 x 3 min; 1 x 10 min) abgespalten. Das Harz wurde je 2 mal mit DMF und Isopropanol gewaschen. Daraufhin wurden 2 mmol Fmoc-Ala mit 3 mmol HOBt und 2.2 mmol DIC in 15 ml DMF mit dem Harz eine Stunde lang inkubiert. Überschüssige Reagentien wurden dann abfiltriert und das Harz je 2 mal mit DMF und Isopropanol gewaschen. Die Vollständigkeit der Umsetzung wurde mit einem Ninhydrintest geprüft und bei eventueller Unvollständigkeit wurde die Kopplung wiederholt. Nach dieser Methode wurden auch die anderen Aminosäuren angekoppelt. Bei Arg wurde die Pmc-Schutzgruppe eingesetzt. Als letzte Aminosäure wurde eine Boc-Aminosäure benutzt. Das Peptidharz wurde mit Methanol und Diethylether gewaschen und im Vakuum getrocknet. Das Harz wurde mit 20 ml einer Mischung aus 90 % TFA und 10 % Ethandithiol 1 Stunde bei 35°C behandelt. Das gelöste Peptid wurde in Ether kristallisiert und an ^{R}Sephadex-G25 in 0.5 % Essigsäure chromatographiert. Der Peptidpool wurde gefriergetrocknet und war laut HPLC zu 95 % rein. Die Aminosäureanalyse zeigte einen Peptidgehalt von 68 %.

### Austestung:

Zu 25 Mikroliter Plasma wurden 100 Mikroliter des Gerinnungsinhibitor (Peptid) unterschiedlicher Konzentration gegeben. Die Umwandlung des Fibrinogens in Fibrin wurde durch Zugabe von 25 Mikroliter Thrombin (50 IU/ml) ausgelöst. Die Gerinnung des Ansatzes wurde durch Messung der Trübung auf einem Gerinnungsanalysator (ACL 300 mit angeschlossenem Rechner) verfolgt.

In einem zweiten Ansatz wurden zu 100 Mikroliter Plasma jeweils 50 Mikroliter der Peptidlösung und 50 Mikroliter Thrombinlösung zugegeben und wie oben gemessen. Als Eintritt der Gerinnung wurde der Zeitpunkt angesehen, bei dem das Streulicht um den gleichen Betrag angestiegen war, wie es vom Hersteller des Gerätes (Instrumentation Laboratory, Mailand) für die Bestimmung der Prothrombinzeit bei Plasmaproben vorgesehen ist.

Alle angegebenen Peptide sind Peptidamide (R₁=R₂=H). Die angegebenen Werte sind Gerinnungszeiten in Sekunden.

| | | | | | | |
|---|---|---|---|---|---|---|
| Testansatz I: 100 µl Clotinhibitor 25 µl Plasma 25 µl Thrombin | | | | | | |

| **Gerinnungszeiten in Sekunden** | | | | | | |
|---|---|---|---|---|---|---|
| Konzentration des Clotinhibitors | | | | | | |
| mg/ml | 2 | 1 | 0,5 | 0,25 | 0,125 | 0,06 |
| Peptidamid | | | | | | |
| GPRPPP Vergleichsversuch | 592,2 | 498,1 | 201,7 | 76,3 | 28,8 | 21,2 |
| GPRPG | 999,9 | 718,5 | 229,3 | 73,5 | 27,9 | 20,3 |
| GPRPPR Vergleichsversuch | 493,4 | 310,9 | 109,6 | 66,8 | 30,7 | 21,2 |
| GPRPRP Vergleichsversuch | 574,1 | 617,8 | 205,5 | 79,2 | 32,6 | 21,2 |
| GPRPA | 635,9 | 535,0 | 300,5 | 148,5 | 51,6 | 24,1 |
| GPRPD Vergleichsversuch | 430,7 | 166,6 | 45,9 | 22,2 | 20,3 | 21,2 |
| GPRPW Vergleichsversuch | 879,1 | 726,1 | 190,3 | 70,6 | 26,9 | 20,3 |
| GPRPK Vergleichsversuch | 258,1 | 139,9 | 173,2 | 102,0 | 51,6 | 25,0 |
| GPRPS Vergleichsversuch | 115,3 | 114,3 | 83,3 | 41,2 | 23,1 | 20,3 |
| GPRP Vergleichsversuch | 494,3 | 181,8 | 48,8 | 23,1 | 20,3 | 20,3 |
| GPRPP Vergleichsversuch | 699,5 | 425,9 | 228,3 | 73,5 | 28,8 | |
| GPRPV Vergleichsversuch | 174,2 | 225,5 | 148,5 | 83,0 | 32,6 | |
| GPRPI Vergleichsversuch | 785,0 | 968,4 | 181,8 | 57,3 | 25,9 | |
| GPRPF Vergleichsversuch | 482,9 | 196,0 | 88,7 | 33,6 | 21,2 | |
| GPRPAG Vergleichsversuch | 201,7 | 191,3 | 142,8 | 84,9 | 33,6 | |
| GLRPG Vergleichsversuch | 25,0 | 25,0 | 25,0 | 24,1 | 24,1 | |

| | | | | | | |
|---|---|---|---|---|---|---|
| Testansatz II: 50 µl Clotinhibitor 100 µl Plasma 50 µl Thrombin | | | | | | |

| **Gerinnungszeiten in Sekunden** | | | | | | |
|---|---|---|---|---|---|---|
| Konzentration des Clotinhibitors | | | | | | |
| mg/ml | 2 | 1 | 0,5 | 0,25 | 0,125 | 0,06 |
| Peptidamid | | | | | | |
| GPRPPP Vergleichsversuch | 316,7 | 61,1 | - | 19,3 | 19,3 | 20,3 |
| GPRPG | 126,7 | 43,1 | 20,3 | 18,4 | 19,3 | 19,3 |
| GPRPPR Vergleichsversuch | 89,6 | 32,6 | 19,3 | 18,4 | 19,3 | 19,3 |
| GPRPRP Vergleichsversuch | 106,7 | 36,4 | 19,3 | 18,4 | 19,3 | 19,3 |
| GPRPA | 448,7 | 83,9 | 25,9 | 19,3 | 19,3 | 19,3 |
| GPRPD Vergleichsversuch | 350,9 | 48,8 | 47,8 | 19,3 | 20,3 | 20,3 |
| GPRPW Vergleichsversuch | 85,8 | 25,0 | 19,3 | 19,3 | 19,3 | 20,3 |
| GPRPK Vergleichsversuch | 113,4 | 35,5 | 19,3 | 18,4 | 19,3 | 19,3 |
| GPRPS Vergleichsversuch | 77,3 | 25,0 | 18,4 | 18,4 | 19,3 | 20,3 |
| GPRP Vergleichsversuch | 31,7 | 19,3 | 19,3 | 19,3 | 20,3 | 20,3 |
| GPRPP Vergleichsversuch | 239,7 | 60,2 | 21,2 | 18,4 | 19,3 | 19,3 |
| GPRPV Vergleichsversuch | 168,4 | 43,1 | 20,3 | 18,4 | 19,3 | |
| GPRPI Vergleichsversuch | 93,4 | 27,9 | 19,3 | 18,4 | 20,3 | |
| GPRPF Vergleichsversuch | 47,8 | 20,3 | 18,4 | 19,3 | 19,3 | |
| GPRPAG Vergleichsversuch | 195,1 | 49,7 | 20,3 | 18,4 | 19,3 | |
| GLRPG Vergleichsversuch | 21,2 | 21,2 | 21,2 | 20,3 | 21,2 | |

| | | | | | |
|---|---|---|---|---|---|
| Testansatz III: 20 µl Clotinhibitor mit Thrombin 130 µl Plasma | | | | | |

| **Gerinnungszeit in Sekunden** | | | | | |
|---|---|---|---|---|---|
| Konzentration des Clotinhibitors | | | | | |
| mg/ml | 10 | 5 | 2,5 | 1,25 | 0,625 |
| Peptidamid | | | | | |
| GPRPPP Vergleichsversuch | 43,1 | 85,8 | 35,5 | 20,3 | 18,4 |
| GPRPG | 656,8 | 104,8 | 40,2 | 19,3 | 18,4 |
| GPRPPR Vergleichsversuch | 209,3 | 70,6 | 28,8 | 19,3 | 18,4 |
| GPRPRP Vergleichsversuch | 323,3 | 77,3 | 28,8 | 18,4 | 18,4 |
| GPRPA | 999,9 | 272,9 | 76,3 | 25,0 | 18,4 |
| GPRPD Vergleichsversuch | 101,0 | 24,1 | 18,4 | 18,4 | 17,4 |
| GPRPW Vergleichsversuch | 155,2 | 38,3 | 19,3 | 18,4 | 18,4 |
| GPRPK Vergleichsversuch | 575,1 | 114,3 | 41,2 | 20,3 | 18,4 |
| GPRP Vergleichsversuch | 926,6 | 86,7 | 22,2 | 18,4 | 18,4 |
| GPRPP Vergleichsversuch | 327,7 | 97,2 | 37,3 | 20,3 | 18,4 |
| GPRPV Vergleichsversuch | 69,7 | 109,6 | 43,1 | 20,3 | 18,4 |
| GPRPI Vergleichsversuch | 448,7 | 107,7 | 32,6 | 19,3 | 18,4 |
| GPRPF Vergleichsversuch | 261,6 | 59,2 | 20,3 | 18,4 | 18,4 |
| GPRPAG Vergleichsversuch | 22,2 | 71,6 | 45,0 | 20,3 | 18,4 |
| GLRPG Vergleichsversuch | 18,4 | 18,4 | 18,4 | 18,4 | 18,4 |

## Patentansprüche

1. Peptidamide der allgemeinen Formel I
GPRP-X-NH₂ I
wobei G für die Aminosäure Glycin, P für die Aminosäure L-Prolin, R für die Aminosäure L-Arginin und X für Alanin oder Glycin steht.

2. Peptidamide nach Anspruch 1, dadurch gekennzeichnet, daß die Aminosäuren in der L-Form vorliegen.

3. Verfahren zur Herstellung eines Peptidamids nach Anspruch 1, dadurch gekennzeichnet, daß geschützte Aminosäuren, Aminosäurederivate oder Peptidsegmente in Lösung oder an einer Festphase aneinander gekoppelt werden und durch Abspaltung der Schutzgruppen sowie im Falle einer Festphase durch Abspaltung vom Trägerharz Peptide gemäß Struktur (I) erhalten werden.

4. Peptidamid nach Anspruch 1 zur Verwendung als Arzneimittel.

5. Verwendung eines Peptids nach Anspruch 1 für diagnostische Zwecke oder als Diagnostikum.

## Claims

1. A peptide amide of the formula I
**GPRP-X-NH**_{**2**} **I**
where G is the amino acid glycine, P is the amino acid L-proline, R is the amino acid L-arginine and X is alanine or glycine.

2. A peptide amide as claimed in claim 1, wherein the amino acids are present in the L form.

3. A process for the preparation of a peptide amide as claimed in claim 1, which comprises protected amino acids, amino acid derivatives or peptide segments being coupled to one another in solution or on a solid phase, and peptides according to structure (I) being obtained by cleaving off the protecting groups and, in the case of a solid phase, by cleaving off from the support resin.

4. A peptide amide as claimed in claim 1 for use as medicament.

5. The use of a peptide as claimed in claim 1 for diagnostic purposes or as diagnostic agent.

## Revendications

1. Peptidamide de formule générale I
GPRP-X-NH₂ I
dans laquelle G est l'acide aminé glycine, P est l'acide aminé L-proline, R est l'acide aminé L-arginine, X est l'alanine ou la glycine.

2. Peptidamide selon la revendication 1, caractérisé en ce que les acides aminés sont sous la forme L.

3. Procédé pour la préparation d'un peptidamide selon la revendication 1, caractérisé en ce qu'on couple les uns aux autres, en solution ou en phase solide, des acides aminés, des dérivés d'acides aminés ou des fragments peptidiques protégés et ' en ce qu'on obtient, par élimination des groupes protecteurs ainsi que, dans le cas d'une phase solide, par scission de la résine support, des peptides de structure (I).

4. Peptidamide selon la revendication 1 pour une utilisation en tant que médicament.

5. Utilisation d'un peptide selon la revendication 1 à des fins diagnostiques ou comme agent de diagnostic.
